Europäisches Patentamt

European Patent Office

Office européen des brevets

(11) Publication number: 0 386 285 A1

# EUROPEAN PATENT APPLICATION

(21) Application number: 89103995.0

(51) Int. Cl.5: A61B 17/10

(22) Date of filing: 07.03.89

(43) Date of publication of application:
12.09.90 Bulletin 90/37

(84) Designated Contracting States:
AT BE CH DE ES FR GB GR LI LU NL SE

(71) Applicant: RICERCHE BIOMEDICHE S.R.L.
Via Serbelloni 4
I-20122 Milano(IT)

(72) Inventor: Palmieri, Beniamino
Via Bisi, 125
I-41100 Modena(IT)
Inventor: Garlati, Rodolfo
Via Belvedere 11/C
I-22057 Olginate (Como)(IT)

(74) Representative: Münzhuber, Robert,
Dipl.-Phys. et al
Patentanwalt Rumfordstrasse 10
D-8000 München 5(DE)

(54) Improvements in automatic mechanic suturing guns.

(57) The suturing gun is of the type having the usual stitch applying device and comprises pliers (P) for clamping the wound margins arranged close the stitch outlet (8), a control element (7) for actuating the pliers (P) and arranged outside the gun, in a portion easily accessible by the surgeon hand grasping the gun and a driving system (11,12, 13,17,20,21) for transmitting the movement of the control element (7) to the pliers (P).

The suturing gun further comprises housing means (31) for containing and retaining one or more gauze tampons, first dispensing means (33) for containing and dispensing a disinfectant liquid or the like and second dispensing means (37,38,39,40,41) for containing and dispensing a suture adhesive.

FIG.6

EP 0 386 285 A1

## IMPROVEMENTS IN AUTOMATIC MECHANIC SUTURING GUNS

The present invention generally relates to suturing devices and, more particularly, an improved suturing gun capable to exhibit much greater performances than the suturing guns available in the commerce.

In the surgery field mechanical suturing devices for the skin are already known, which generally are produced in the shape of a gun. In the use a suturing gun of this kind is grasped by one hand of the surgeon, while his other hand operates a forceps in order to clamp the margins of the wounded skin together and maintain them in this clamped position so that the stitches delivered by the suturing gun can grip both the wound margins, in order to perfectly fit them together. This procedure is very toilsome for the surgeon and often, to facilitate this operation, the mechanical suturing device is grasped by the right hand of the surgeon and the clamping of the skin margins is carried out by an assistent who operates a pair of toothed forceps and so holds the wound margins clamped until the stitches have been applied. The latter solution, if on one hand facilitates the surgeon task, on the other hand requires the presence of two operators and therefore increases the costs of the whole operation.

It is also known that during the suture operation the surgeon needs, for the immediate use, to have at his disposal gauze tampons to plug the wound, as well as disinfectant liquids and suture adhesives, which assist to maintain the wound disinfected during the suture operation and sutured after the suture operation. The application of these products requires again the presence of a second operator during the suturing operation made by the surgeon, which operator must give the surgeon gauze tampons, disinfecting liquid and/or adhesive on request.

The present invention aims at obviating all the above mentioned disadvantages by providing a new and improved suturing gun which permits the suturing operation of a wound to be carried out by the surgeon with one hand only, thereby obtaining the advantages of better clamping the wound margins, facilitating the suturing operation and requiring lesser time for carrying out syncronous interventions.

It is another object of the present invention to provide an improved suturing gun which permits the suturing operation of a wound to be carried out by the surgeon without the necessity of resorting to an assistant.

It is another object of the present invention to provide an improved suturing gun which permits the whole suturing operation of a wound to be carried out by the surgeon without resorting to an assistant giving the surgeon disinfectant liquids, adhesives and gauze tampons in the course of the suture operation.

These and other objects which will be more apparent in the course of the description are attained, according to the invention, by a suturing gun which is characterized in that, in addition to the conventional stitch applying device, it comprises:

- pliers for clamping the wound margins, arranged close the stitch outlet;
- a control element for actuating said pliers means and arranged outside the gun, in a position easily accessible by the surgeon hand;
- driving means for transmitting the movement of the control element to said clamping means;
- housing means for containing and retaining gauze tampons;
- first dispensing means for containing and dispensing a disinfectant liquid; and
- second dispensing means for containing and dispensing a suture adhesive.

The present invention will be now described in more detail in connection to a preferred embodiment thereof, given only by way of example and therefore not intended in a limiting sense, illustrated in the accompanying drawings, wherein:

Fig. 1 is a perspective view of the suturing gun according to the present invention, in the rest position;

Fig. 2 is a view similar to Fig. 2, however with the forceps closed in the position of approach of the wound margins;

Fig. 3 is a view similar to Fig. 2, with the gun in the active position to deliver the suture stitches;

Fig. 4A is a side view of the driving means for actuating the forceps, with the forceps being in open position;

Fig. 4B is a plan view of the driving system for actuating the forceps of Fig. 4A;

Fig. 5A is a side view of the driving system for actuating the forceps, with the forceps being in closed position;

Fig. 5B is a plan view of the driving system for actuating the forceps of Fig. 5A; and

Fig. 6 is a side elevation view of the suturing gun according to the present invention, partially sectioned to show the disinfectant container, the adhesive pump and the container for the gauze tampons.

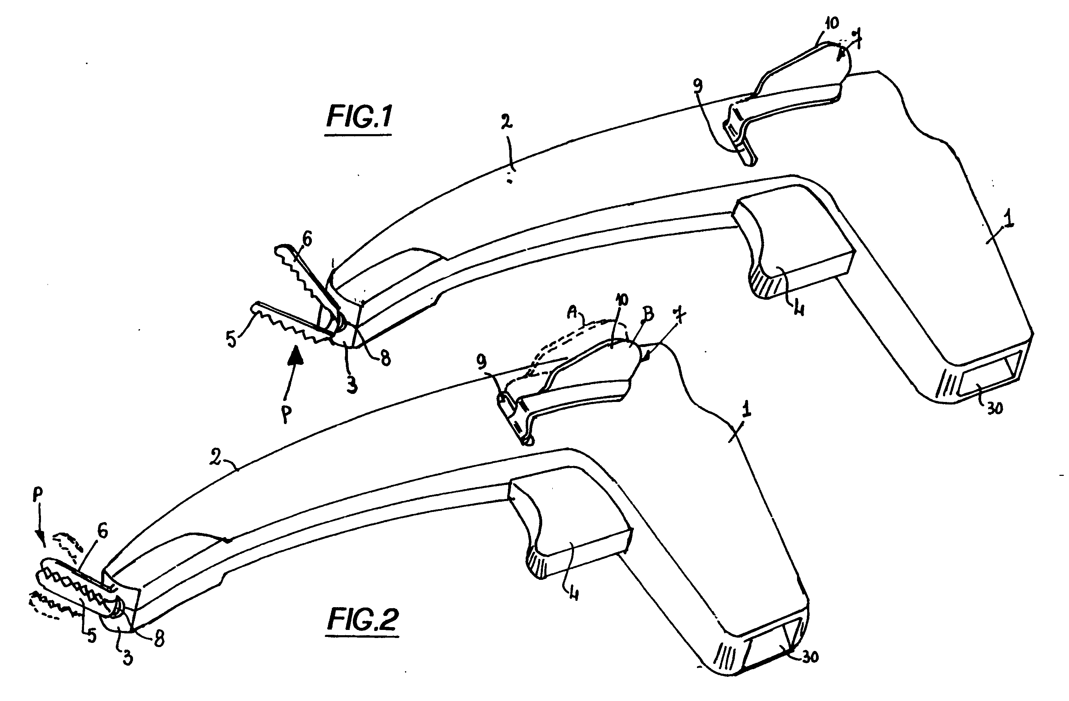

Referring now to Figs. 1 to 3, there is shown the mechanical automatic suturing gun according to the invention which comprises a grip 1, a body 2, a tip 3, a trigger 4 as usual in the suturing guns.

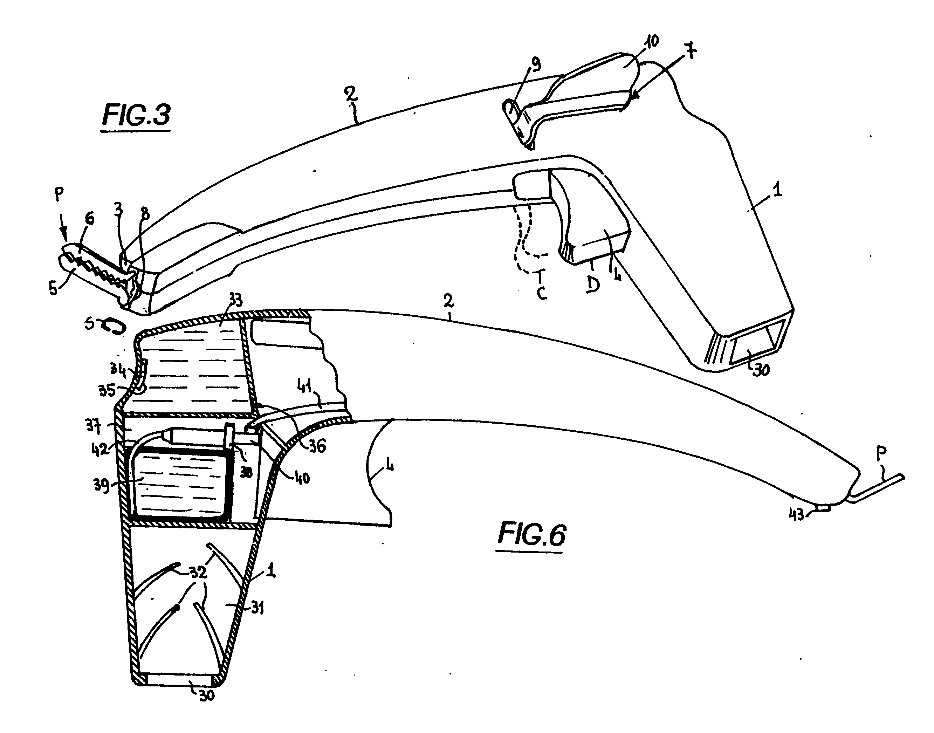

At the tip 3 from which, by operating the trigger 4, the suture stitches are delivered through the slit 8 (Fig. 3) pliers are applied, which are generally designated with P and which are formed of a pair of toothed jaws 5,6 pivotally connected to each other and controlled, through a mechanical driving system which will be described later, by a control element 7 arranged outside the gun body 2, above the trigger 4. The jaws 5,6 of the pliers are slanted with respect to the longitudinal axis of the gun.

In Fig. 1 the suturing gun is shown in a rest position in which the jaws 5,6 of the pliers P are open and the trigger 4 and the control element 7 are in the rest position.

In Fig. 2 the suturing gun is shown in a position in which it clamps the wound margins, wherein the jaws 5,6 of the pliers P have been closed by the control element 7 which is moved from the position A to the position B, while the trigger 4 is again in the rest position.

In Fig. 3 the suturing gun is shown in the suturing position with the jaws 5,6 of the pliers P being in a closed position in which they retain always the wound margins therebetween and with the trigger 4 being moved from the position C to the position D, so that the suture stitch S is applied to the approached wound margins, thereby suturing the wound. The control element 7 penetrates the body 2 of the suturing gun through a slot 9 and is provided at the outer end thereof with a lug 10 intended to act as a grip element for a surgeon finger.

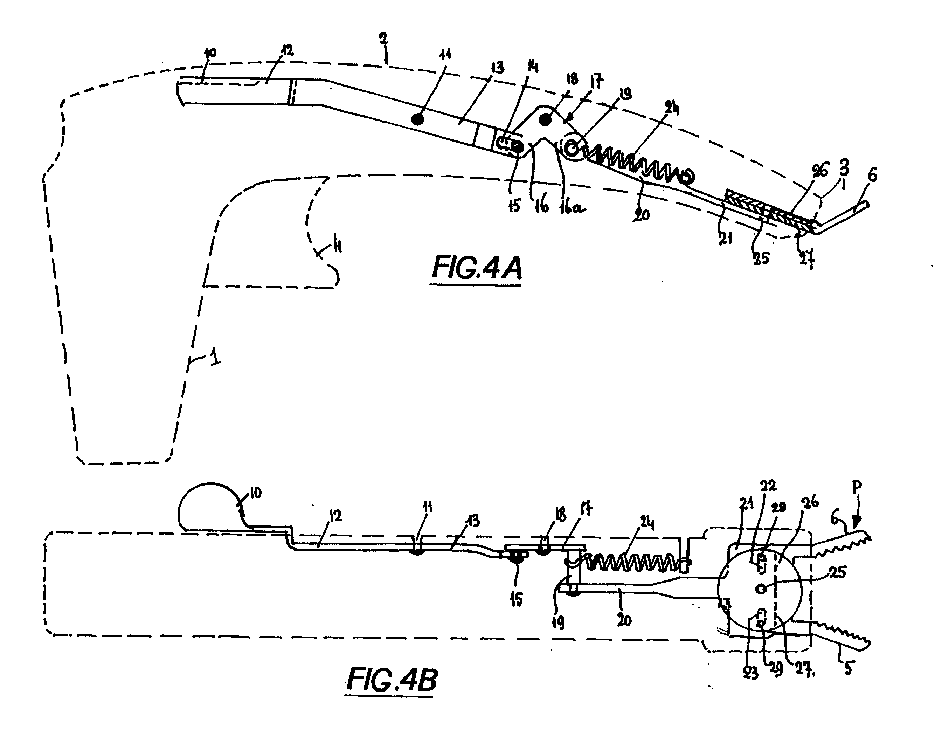

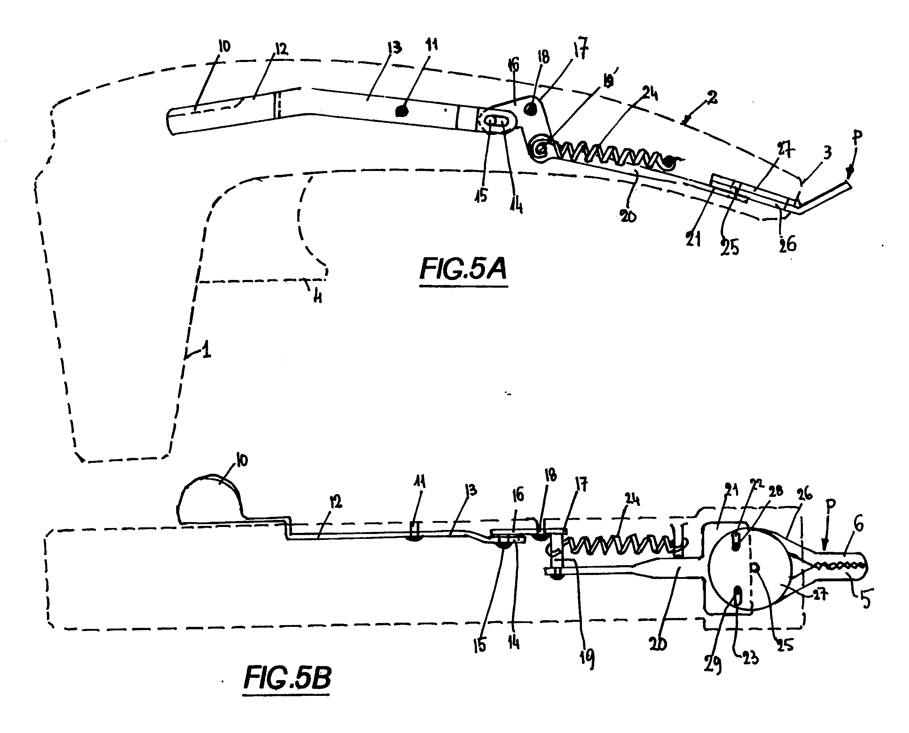

In Figs. 4A,4B; 5A,5B there is shown in a side view and in a plan view, respectively the driving system for actuating the pliers P, in Figs. 4A and 4B the pliers being in an open position and in Figs. 5A and 5B the pliers being in a closed position. The control element 7 is formed of a two-arm lever which is pivotally mounted on the pivot 11, and the arm 12 of which extends outwardly and is provided with the lug 10, whereas the other arm 13 of the lever is provided at its end with a slot 14 adapted to receive a pin 15 fastened on an arm 16 of a bell crank 17 pivotally mounted on a pivot 18. The other arm 16a of the bell crank 17 is also provided with a pin 19 to which a tie rod 20 is connected, this tie rod 20 ending at its free end in a plate 21 provided with a pair of transversal and aligned slots 22 and 23. Connected to the pin 19 of the bell crank 17 is also one end of a tension coil spring 24 which is fastened at the other end to the gun body 2.

The jaws 5,6 of the pliers, as already said, are pivotally connected to each other on the pivot 25 through portions 26 and 27, respectively having substantially the shape of a disc which forms the pivot portion of the pliers. The disc 26 is provided at its lower end with a post 28 adapted to engage in the slot 22 of the plate 21 of the tie rod 20 and the disc 27 is provided at its lower portion with a post 29 adapted to engage in the slot 23 of the plate 21 of the tie rod 20.

When the control element 7 is lowered acting on the lug 10, the lever arm 13 rotates in a counterclockwise direction, so that the bell crank will rotate in a clockwise direction and the tie rod 20 will be pulled inwardly in the gun, against the force of the tension spring 24. This rearwardly movement of the tie rod 20 and therefore the plate 21 will cause the posts 28,29 of the discs forming the pivot portion of the pliers P to move in the respective slots 22 and 23 from the outer position to the inner position, so that the disc 26 will rotate in clockwise direction and disc 27 will rotate in counterclockwise direction (Fig. 4B).This rotatory movement will cause the jaws 5,6 of pliers P to swing until they will come in the closed position (Fig. 5B). In this position the jaws 5,6 will retain the wound margins clamped.

When the control element 7 is released, the tension spring 24 will return all the components of the driving system just described to their initial positions, i.e. in the rest position of the suturing gun (see Fig. 1), in which the jaws 5,6 of the pliers P are again open.

Fig. 6 illustrates the suturing gun in a longitudinal section, with the driving system for actuating the pliers being omitted. This Figure illustrates the three other features offered by the suturing gun and arranged in the grip 1 thereof.

More particularly, in the bottom of the grip 1 a hole 30 is provides which opens in a lower chamber 31 having resilient tongues 32 arranged at least along the opposite walls thereof. The function of this chamber 31 is to receive one or more gauze tampons, not shown, whereas the function of the resilient tongues 32 is to retain these gauze tampons in the chamber. The gauze tampons are inserted in the hole 30 of the chamber 31 by hand, preferably at the time that the suturing guns is to be used.

In the upper portion of the gun grip 1 another chamber 33 is provided, which is intended to receive a disinfectant liquid or the like. More particularly, the chamber 33 has an inlet hole 34 on the rear wall of the gun, which is provided with a check valve 35 and, on the wall opposite that in which the inlet hole 34 is provided, a capillary hole 36 is provided, the function of which is to let the disinfectant liquid to flow by gravity when the suturing gun is in the vertical or use position. This disinfectant liquid passes from the capillary hole 36, through the gun body 2 and flows from the slot 8 from which the stitches S are dispensed, thereby disinfecting the wound margins during the suturing operation. The chamber 33 can be filled with disinfectant liquid by inserting through the inlet hole 34 the needle of a syringe, which opens the check valve 35 and then injecting in the chamber 33 the disinfectant liquid. Once the syringe needle has been removed from the hole 34 the check valve 35 automatically closes.

In the grip 1, between the upper chamber 33 and the lower chamber 31 a further chamber 37 is provided, in which a container 39 of suture adhesive and a small suction pump 38 are arranged, said suction pumpe 38 being connected to the container 39 by a flexible tube 42 dipped in the container 39. The piston 40 of the suction pump 38 abuts the rear edge of the gun trigger 4. The delivery side of the pump 38 communicates, through a flexible tube 41, with a sprout 43 placed close the slot 8 from which the suture stitches S are dispensed. With this arrangement, when the trigger 4 is pulled to deliver a suture stitch S from the outlet slot 8 and its application on the wound margins the suction pump 38 is simultaneously actuated, which, by sucking the suture adhesive from the container 39 through the flexible tube 42 and delivering it in the flexible tube 41, causes it to flow from the opening 43 on the sutured wound.

By means of this suturing gun the surgeon can clamp the wound margins by acting on the control element 7 and then apply the stitches for suturing the wound margins so clamped by pulling the trigger 4 and this by one hand only. In the meantime, the wound to be sutured is subjected to the action of the disinfectant liquid or the like which flows from the capillary hole 36 and during the stitch application also the suture adhesive is dispensed on the wound.

From the foregoing it is readily apparent that the suturing gun according to the invention offers the following great advantages:

1) the surgeon has one hand free for helping himself in the suturing operation, what means that he needs no more an assistant as with the known suturing guns;

2) the gun is provided with pliers for clamping the wound margins, which pliers can be actuated always by the same hand of the surgeon and permit the suturing operation to be extremely facilitated;

3) the gun is provided with a housing for gauze tampons so that they are always at reach of the surgeon during the suturing operation and can be taken by the free hand of the surgeon for being then used for tamponing the wound;

4) the gun is provided with a small reservoir of disinfectant liquid or the like, what eliminates the requirement of a further operator which gives or perhaps applies the disinfectant liquid on the wound;

5) the gun is provided with a small reservoir for the suture adhesive, which can be pumped on the wound as the gun trigger is pulled.

While the present invention has been described in connection to a preferred embodiment thereof only, it is apparent that those skilled in the art can make thereto various modifications and changes without departing from the scope of the invention. In particular, the driving system for actuating the gripper could be made in a manner other than that illustrated and the suturing gun according to the invention could have one or two or all the additional features here illustrated.

## Claims

1) Automatic mechanical suturing gun of the type in which the trigger causes the delivery and the application of the suture stitches on the wound to be sutured, characterized in that, in addition to the conventional stitch applying device, it comprises:
- pliers for clamping the wound margins, arranged close the stitch outlet;
- a control element for actuating said pliers means and arranged outside the gun, in a position easily accessible by the surgeon hand;
- driving means for transmitting the movement of the control element to said pliers;
- housing means for containing and retaining gauze tampons;
- first dispensing means for containing and dispensing a disinfectant liquid; and
- second dispensing means for containing and dispensing a suture adhesive.

2) Suturing gun according to claim 1, characterized in that the pliers are formed of a pair of toothed jaws and the driving means for actuating them comprise a linkage arranged inside the gun and connected to said control element.

3) Suturing gun according to claim 2, characterized in that the linkage actuating the pliers comprise a two arm lever, one arm of which is connected to said control element and the other arm of which is pivotally connected to an arm of a bell crank, the other arm of which is pivotally connected to a tie rod controlling the toothed jaws of the pliers.

4) Suturing gun according to claim 3, characterized in that the pliers jaws are inclined with respect to the pivoting portion of the pliers, said pivoting portion being formed of a pair of discs pivotally connected to each other, each of said discs being provided with a projecting post, said posts engaging in slots provided in the free end portion of said tie rod so that, when said tie rod is pulled by the linkage, the engagement of said posts in the slots of the rod end portion causes the pliers jaws to be rotated in the closed position, a return spring being provided for returning the above components in their start positions.

5) Suturing gun according to claim 1, characterized in that said housing means for containing and retaining the gauze tampons is formed of a chamber provided in the gun grip and having tongues for retaining the gauze tampons.

6) Suturing gun according to claim 1, characterized in that said first dispensing means for containing and dispensing a disinfectant liquid or the like is formed of a reservoir arranged in the gun grip and capable of delivering the disinfectant liquid on the wound during the suturing operation, this delivery occurring by gravity when the suturing gun is in the use position.

7) Suturing gun according to claim 6, characterized in that the disinfectant liquid reservoir has an inlet hole provided with a check valve and a capillary hole communicating with the outlet of the suture stitches.

8) Suturing gun according to claim 1, characterized in that said second dispensing means for containing and dispensing a suture adhesive is formed of a reservoir for the suture adhesive, a pump connected to said reservoir and arranged so as to be actuated by the gun trigger in order to dispense this adhesive on the wound at the time that the trigger is pulled for applying the suture stitch.

9) Suturing gun according to claim 8, characterized in that the pump is a suction pump of the type usually employed in the known liquid dispensers.

FIG.1

FIG.2

FIG.3

P
6
3
8
5
5
2
9
10
7
1
C
D
4
30

FIG.6

33
34
35
37
42
39
36
38
40
41
2
4
P
43
32
1
31
30

EP 0 386 285 A1

FIG.4A

FIG.4B

_FIG.5A_

_FIG.5B_

EP 0 386 285 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| A | WO-A-8 505 025 (PUCHY) <br> * Abstract; page 11, lines 12-25; figures 1-5,32; claims 5,11 * <br> --- | 1-4 | A 61 B 17/10 |
| A | US-A-4 123 982 (BESS, Jr. et al.) <br> * Column 3, lines 40-58; figure 7 * <br> --- | 3 | |
| A | EP-A-0 094 752 (KAUFMAN) <br> ----- | | |

|  |  |  | TECHNICAL FIELDS SEARCHED (Int. Cl. 5) |
|---|---|---|---|
|  |  |  | A 61 B |

The present search report has been drawn up for all claims

| Place of search | Date of completion of the search | Examiner |
|---|---|---|
| THE HAGUE | 31-10-1989 | SANCHEZ Y SANCHEZ J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)